# EUROPEAN PATENT SPECIFICATION

(11) **EP 0 341 607 B1**
(45) Date of publication and mention of the grant of the patent: **20.07.1994**
(21) Application number: 89108159.8
(22) Date of filing: 05.05.1989
(51) Int. Cl.: C07K 7/00, A61K 37/02

(54) **Anticoagulant peptide alcohols**
Antikoagulierende Peptid-Alkohole
Peptides alcools anticoagulants

(30) Priority: 10.05.1988 US 192409
(43) Date of publication of application: 15.11.1989
(73) Proprietor: MERRELL DOW PHARMACEUTICALS INC., Cincinnati Ohio 45215-6300 (US)
(72) Inventor: Krstenansky, John L., Cincinnati, OH 45208 (US)
(74) Representative: VOSSIUS & PARTNER

(56) References cited:
- EP-A- 0 158 986
- EP-A- 0 276 014
- CHEMICAL ABSTRACTS, vol. 111, 1989, Columbus, OH (US); J.L. KRSTENANSKY et al., p. 18, no. 70327w#

## Description

This invention relates to novel peptide alcohols which are useful anticoagulant agents.

Anticoagulants are useful therapeutic agents in the pharmacological treatment of, for example, acute deep venous thrombosis, pulmonary embolism, acute arterial embolization of the extremities, myocardial infarction, and disseminated intravascular coagulation. Prophylactic administration of anticoagulants is believed to prevent a recurrence of embolism in patients with rheumatic or arteriosclerotic heart disease and to prevent certain thromboembolic complications of surgery. Administration of anticoagulants has also been indicated in the treatment of coronary artery and cerebrovascular disease. Arterial thrombosis, particularly in arteries supplying the heart muscle and brain, is a leading cause of death.

Hirudin is a 65 residue polypeptide isolated from the salivary glands of leeches. It is an anticoagulant agent, which is a thrombin specific inhibitor. Although quite potent, clinical use of hirudin isolated from leech extracts seems unlikely because of its limited quantity, expense, and allergic reactions which commonly follow administration of any foreign protein of this size.

Applicants have discovered a specific region of hirudin that is responsible, at least in part, for its anticoagulant activity. This region has been chemically synthesized and certain of its analogs appear to bind to the recognition site of thrombin but not the enzymatic cleavage site which is spatially separate. Binding of the synthetic peptides competitively prevents binding of the fibrinogen to the recognition site of thrombin, a prerequisite to fibrin production and clot formation. Applicants have now prepared certain reduced derivatives of this peptide. The carboxylic acid function of these new derivatives has been reduced to the corresponding alcohol functionality. The peptide alcohols of this invention possess significant anticoagulant activity and their unusual ability to bind only to the recognition site without binding to the cleavage site of thrombin may allow for a scientifically interesting and therapeutically significant adjunct to anticoagulant therapy. Moreover, the presence of the alcohol function may provide for enhanced potency and extended duration of action.

Peptide derivatives of the formula
X-A₁-A₂-A₃-A₄-A₅-A₆-A₇-A₈-A₉-A₁₀-ol
wherein
- X: is an amino terminal residue selected from hydrogen, one or two alkyl groups of from 1 to 6 carbon atoms, one or two acyl groups of from 2 to 10 carbon atoms, carbobenzyloxy or t-butyloxy carbonyl;
- A₁: is a bond or is a peptide containing from 1 to 11 residues of any amino acid;
- A₂: is Phe, Subphe, β-(2- and 3-thienyl)alanine, β-(2-and 3-furanyl)alanine, β-(2-, 3-, and 4-pyridyl)alanine, β-(benzothienyl-2- and 3-yl)alanine, β-(1- and 2-naphthyl)alanine, Tyr or Trp;
- A₃: is Glu or Asp;
- A₄: is any amino acid;
- A₅: is Ile, Val, Leu, Nle, or Phe;
- A₆: is Pro, Hyp, 3,4-dehydroPro, thiazolidine-4-carboxylate, Sar, NMePgl or D-Ala;
- A₇: is any amino acid;
- A₈: is any amino acid;
- A₉: is a lipophilic amino acid selected from Tyr, Met, Trp, Phe, Leu, Nle, Ile, Val, Cha and Pro or is a dipeptide containing at least one of these lipophilic amino acids; and
- A₁₀-ol: is a reduced peptide fragment containing from zero to five residues of any amino acid wherein the carbon terminal amino acid is reduced to its alcohol derivative;
are useful anticoagulant agents.

The following common abbreviations of the amino acids are used throughout this specification:
- Ac -: acetyl
- Ala (or A) -: alanine
- DAla (or a) -: D-alanine
- Arg (or R) -: arginine
- Asn (or N) -: asparagine
- Asp (or D) -: aspartic acid
- pClPhe -: para-chloro-phenylalanine
- Cha-: cyclohexylalanine
- Cys (or C) -: cysteine
- 3,4-dehydroPro -: 3,4-dehydroproline
- Gly (or G) -: glycine
- Glu (or E) -: glutamic acid
- D-Glu (or e) -: D-glutamic acid
- Gln (or Q) -: glutamine
- Glt -: glutaryl
- His (or H) -: histidine
- Hyp -: hydroxyproline
- Ile (or I) -: isoleucine
- Leu (or L) -: leucine
- Lys (or K) -: lysine
- Mal -: maleyl
- Met (or M) -: methionine
- NmePgl -: N-methyl-phenylglycine
- Npa -: β-(naphthyl)alanine
- pNO₂Phe -: para-nitro-phenylalanine
- Nle -: norleucine
- Orn -: ornithine
- pSubPhe -: para substituted phenylalanine
- Phe (or F) -: phenylalanine
- Pgl -: phenylglycine
- Pro (or P) -: proline
- Sar -: sarcocine (N-methylglycine)
- Ser (or S) -: serine
- SubPhe -: ortho, meta, or para, mono- or di- substituted phenylalanine
- Suc -: succinyl
- Thr (or T) -: threonine
- Trp (or W) -: tryptophan
- Tyr (or Y) -: tyrosine
- Val (or V) -: valine
By the expression "a reduced peptide fragment containing from one to five residues of any amino acid wherein the carbon terminal amino acid is reduced to its alcohol derivative" applicants intend that the carbon atom of the carboxylic acid group of the carbon terminal amino acid is replaced with a hydroxymethyl group, -(CH₂)OH. While the A₁₀ group of the polypeptides of this invention may contain up to five amino acids, it is intended that only the carbon terminal amino acid be reduced to its corresponding alcohol derivative. Indeed, it is of course not possible that any other but the carbon terminal amino acid be so reduced because peptidic linkage would be impossible. Of course, in those instances wherein A₁₀ is a single amino acid, this single acid is the acid reduced to its alcohol derivative. The structural formula for such reduced amino acids can be represented as: 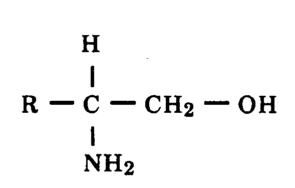
wherein R is the characteristic group of each amino acid. For example, R in the case of glycine is a hydrogen, in the case of alanine is a methyl, in the case of valine is an isopropyl, in the case of phenylalanine is a benzyl, and in the case of cysteine is a mercaptomethyl. The reduced form of proline is an alcohol of the following structural formula. 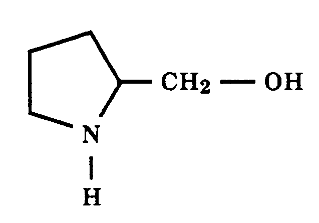
Herein, an amino acid which is reduced to its alcohol derivative will be abbreviated using either the three-letter (or other shortened form) or one-letter code followed by "-ol", for example, "Ala-ol" or "A-ol" means an alanine wherein the carboxylic acid moiety has been reduced to the corresponding alcohol and "Leu-ol" or "L-ol" means a leucine wherein the carboxylic acid moiety has been reduced to the corresponding alcohol.

An alkyl group and the alkyl portion of an alkoxy group is taken to include straight, branched, or cyclic alkyl groups, for example, methyl, ethyl, propyl, isopropyl, butyl, isobutyl, tert.-butyl, pentyl, isopentyl, sec-pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl succinyl, maleyl, and glutaryl. A halogen group is a fluoro, chloro, bromo or iodo group.

The term "any amino acid" as used herein does not purport to include any carboxylic acid having an amino substitutent, but rather is used as it is commonly used by those skilled in the art of polypeptide derivatives and includes the naturally occurring amino acids as well as other "non-protein" α-amino acids commonly utilized by those skilled in the peptide chemistry arts when preparing synthetic analogs of naturally occurring peptides. The naturally occurring amino acids are glycine, alanine, valine, leucine,isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of "non-protein" α-amino acids are norleucine, norvaline, alloisoleucine, homoarginine, thiaproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanines substituted at the ortho, meta, or paraposition of the phenyl moiety with one or two of the following, a (C₁-C₄) alkyl, (C₁-C₄) alkoxy, halogen, or nitro groups or substituted with a methylenedioxy group, β-2- and 3-thienylalanine, β-2- and 3-furanylalanine, β-2-, 3-, and 4-pyridylalanine, β-(benzothienyl-2- and 3-yl)alanine, β-(1- and 2-naphthyl)alanine, O-alkylated derivates of serine, threonine, or tyrosine, S-alkylated cysteine, the O-sulfate ester of tyrosine, 3,5-diiodotyrosine and the D-isomers of the naturally occurring amino acids.

The term "lipophilic amino acid" includes Tyr, Phe, Leu, Met, Nle, Ile, Val, His and Pro.

The natural amino acids, with the exception of glycine, contain a chiral carbon atom. Unless otherwise specifically indicated, the optically active amino acids, referred to herein, are of the L-configuration. For example, any of the amino acids of the A₁ or A₁₀ group can be of the D- or L-configuration. As is customary, the structure of peptides written out herein is such that the amino terminal end is on the left side of the chain and the carboxy terminal end is on the right side of the chain.

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N′-dibenzylethylenediamine, dihydroabietylamine, N-(lower)alkylpiperidine, and any other suitable amine.

As with any generic group of chemical compounds, certain groups are preferred. Applicants prefer those peptide derivatives of formula 1 wherein
- X: is hydrogen, acetyl, or succinyl.
Also preferred are those formula 1 compounds wherein
- A₁: is Thr-Pro-Lys-Pro-Gln-Ser-His-Asn-Asp-Gly-Asp,
-Ser-Thr-Pro-Asn-Pro-Glu-Ser-His-Asn-Asn-Gly-Asp-,
-His-Asn-Asp-Gly-Asp-,
-Asn-Asp-Gly-Asp-,
-Asp-Gly-Asp-,
-Gly-Asp-,
-Asp-, or a bond;
- A₂: Phe, β-2- or 3-thienylalanine, Tyr, Trp, Npa or pClPhe;
- A₃,: Glu;
- A₄,: Glu, Asp, Pro or Ala;
- A₅,: Ile, Leu;
- A₆,: Pro, Sar, D-Ala, Hyp or NMePgl;
- A₇,: Glu, Gln, Asp or Ala;
- A₈,: Glu, Asp or Ala;
- A₉,: Pro, Ala-Tyr, Ala-Cha, Tyr-Cha, Tyr-Leu, Ala-Phe, Tyr-Tyr;
- A₁₀-ol,: Glu-ol, Asn-ol, Pro-ol, Gln-ol, Ala-ol, D-Lys-ol, Lys-ol, D-Asp-ol, Orn-ol or is Asp-Glu-ol.

Especially preferred are those peptide derivatives of formula 1 wherein either
- X: is acetyl and A₁ is Gly-Asp or Asp or
- X: is succinyl and A₁ is a bond and wherein
- A₂: is Phe; β-(2-thienylalanine) or Tyr;
- A₃,: Glu;
- A₄,: Glu or Pro;
- A₅,: Ile;
- A₆,: Pro;
- A₇,: Glu;
- A₈,: Glu or Asp;
- A₉,: Tyr-Leu, Ala-Tyr, Tyr-Tyr, Ala-Phe,Ala-Cha or Pro; and
- A₁₀-ol,: Ala-ol, Gln-ol, Asp-ol, Pro-ol, D-Asp-ol, D-Lys-ol, D-Glu-ol or -Asp-Glu-ol.

The proteins of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of an automated peptide synthesizer. In this procedure, the peptides were constructed on the resin beginning with the penultimate C-terminal, protected amino acid. The resin support employed can be any suitable resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced α-amino protected amino acid. After completion of coupling of the sequence either the Boc protecting group was left in place or it was removed and the N-terminal amino group acylated. Displacement of the protected fragment from the resin was accomplished using the appropriate amino alcohol.

An example of a hydroxymethyl resin is described by Bodanszky et al., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem.Acta. 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and α-chlorobutyryl; (2) aromatic urethan type protecting groups such as benzyloxycarbonyl and substituted benzyloxycarbonyl, such as p-chlorobenzyloxycarbonyl, p-nitrobenzyl- carbonyl, p-bromobenzyloxycarbonyl, p-methoxybenzyloxycarbonyl, 1-(p-biphenylyl)-1-methylethoxycarbonyl, α, α-dimethyl-3,5-dimethoxybenzyloxycarbonyl and benzhydryloxycarbonyl; (3) aliphatic urethan protecting groups such as tert.-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thio urethan type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert.-butyloxycarbonyl.

The selection of an appropriate coupling reagent is within the skill of the art. A particularly suitable coupling reagent where the amino acid to be added is Gln, Asn or Arg is N,N'-diisopropylcarbodiimide and 1-hydroxybenzotriazole. The use of these reagents prevents nitrile and lactam formation. Other coupling agents are (1) carbodiimides (e.g., N,N'-dicyclohexylcarbodiimide and N-ethylN'-(γ-dimethylaminopropylcarbodiimide)); (2) cyanamides (e.g., N,N'-dibenzylcyanamide); (3) ketenimines; (4) isoxazolium salts (e.g., N-ethyl-5-phenyl-isoxazolium-3'-sulfonate); (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N'-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a carbon terminal amino alcohol residue, acetic acid, and dichloromethane (DCM).

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxy-carbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert.-butyl, trityl, benzyl, 2,6-dichlorobenzyl or benzyloxycarbonyl group. The preferred protecting group is benzyl.

These groups can be removed by procedures well known in the art. Typically protecting group removal is done after the peptide chain synthesis is complete but the protecting groups can be removed at any other appropriate time.

The anticoagulant dose of an alcohol peptide derivative of this invention is from 0.2 mg/kg to 250 mg/kg of patient body weight per day depending on the patient, the severity of the thrombotic condition to be treated and the peptide derivative selected. The suitable dose for a particular patient can be readily determined. Preferably from 1 to 4 daily doses would be administered typically with from 5 mg to 100 mg of active compound per dose. The amount of a peptide alcohol of this invention required to inhibit or prevent blood coagulation in an extracorporeal medium such as stored whole blood can be readily determined by those skilled in the art.

Anticoagulant therapy is indicated for the treatment and prevention of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, dogs, cats, rats and mice. Inhibition of blood coagulation is useful not only in anticoagulant therapy of individuals having thrombotic conditions, but is useful whenever inhibition of blood coagulation is desirable, such as to prevent coagulation in stored whole blood and to prevent coagulation in other biological samples for testing or storage.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

### EXAMPLES

This invention is illustrated by the following, nonlimiting examples.

### EXAMPLE 1

### Preparation of Carbon Terminal Peptide Alcohols

The peptides were snythesized using a p-nitrobenzhydrylideneisonitroso resin (oxime resin) prepared by the method of DeGrado and Kaiser (J. Org. Chem. 45, 1295 - 1300 1980)). The oxime resin (0.54 g, 0.52 mmol/0.97 mmol/g substitution) was placed in a vessel of an Applied Biosystems Model 430A peptide synthesizer. Boc-protected amino acids were single coupled sequentially as their symmetrical anhydrides in a two-fold excess. The side chain protection utilized was as folows: Asp(Chx), Glu(Bzl), Tyr(2-BrZ). The programs of the synthesizer were written to give the following protocol for each cycle:
1) Add 1 mmol preformed symmetrical anhydride;
2) Add 5% additional volume of DIEA;
3) Vortex for 1 hour;
4) Drain;
5) Wash (5x/1 min.) with DCM;
6) Wash (1 min.) with 20% TFA in DCM;
7) Treat with 20% TFA in DCM for 15 minutes;
8) Wash with DCM (3x/0.5 min.);
9) Wash(4 min.) with isopropanol
10) Wash (3x/0.5 min.);
11) Wash with isopropanol (4 min.);
12) Wash (3x/0.5 min.) with DCM;
13) Wash (4 min.) isopropanol;
14) Wash (3x/0.5 min.).
After completion of the synthesis, the resin was dried in vacuo. The resin was then treated with 2 equivalents (based on the initial resion substitution) of alaninol and 1 equivalent of acetic acid in DCM at room temperature for 20 hours. The resin was filtered. The filtrates were lyophilized. The residue was treated with liquid HF containing 2% anisole at -5°C for 30 minutes. Upon removal of the HF in vacuo the peptide was extracted with 30% acetonitrile and lyophilized. The residue was purified by preparative HPLC (Dynamax C18 21.4x150mm column) using an acetonitrile/0.1% TFA system. The peptide obtained by this method gave the desired molecular ion peak by FAB-MS and had an amino acid analysis in accordance with the desired peptide. In this way the following peptides having the stated properties were prepared.
1) SucFEPIPEYL-ol
MW 1092.6 FAB-MS (MH)⁺ 1094.0 t_{R} 17.53
E₂₈₀ 1573 peptide content 79.8%

| Glx(2) | Pro(2) | Ile(1) | Tyr(1) | Phe(1) |
|---|---|---|---|---|
| 2.08 | 1.01 | 0.94 | 0.99 | 0.98 |

2) SucFEPIPEEYChaA-ol
MW 1332.7 FAB-MS (MH)⁺ 1333.4 t_{R} 20,2 min.
E₂₈₀ 1755 peptide content 73%

| Glx(3) | Pro(2) | Ile(1) | Tyr(1) | Phe(1) | Cha(1) |
|---|---|---|---|---|---|
| 3.04 | 2.03 | 0.92 | 1.00 | 0.97 | 0.95 |

3) SucFEPIPEEYL-ol
MW 1221.6 FAB-MS (MH)⁺ 1222.8 t_{R} 16.83
E₂₈₀ 1686 peptide content 72.1%

| Glx(3) | Pro(2) | Ile(1) | Tyr(1) | Phe(1) |
|---|---|---|---|---|
| 3.09 | 1.98 | 0.96 | 0.97 | 1.00 |

## Claims (Claims for the following Contracting State(s): AT, BE, CH, DE, FR, GB, IT, LU, LI, NL, SE)

1. An alcohol peptide derivative of the formula
X-A₁-A₂-A₃-A₄-A₅-A₆-A₇-A₈-A₉-A₁₀-ol
wherein
X is a hydrogen, one or two alkyl groups of from 1 to 6 carbon atoms, or one or two acyl groups of from 2 to 10 carbon atoms;
A₁ is a bond or is a peptide fragment containing from one to eleven residues of any amino acid;
A₂ is Phe, SubPhe, β-(2- and 3-thienyl)alanine, β-(2- and 3-furanyl)alanine, β-(2-, 3-, and 4-pyridyl) alanine, β-(benzothienyl-2- and 3-yl)alanine, β-(1- and 2-naphthyl)alanine, Tyr, or Trp;
A₃ is Glu or Asp;
A₄ is any amino acid;
A₅ is Ile, Val, Leu, Nle, or Phe;
A₆ is Pro, Hyp, 3,4-dehydroPro, thiazolidine-4-carboxylate, Sar, NMePgl, or D-Ala;
A₇ is any amino acid;
A₈ is any amino acid;
A₉ is a lipophilic amino acid selected from Tyr, Trp, Phe, Leu, Nle, Ile, Val, Cha and Pro or is a dipeptide containing at least one of these lipophilic amino acids;
A₁₀-ol is a reduced peptide fragment containing from zero to five residues of any amino acid wherein the carbon terminal amino acid is reduced to its alcohol derivative, or -ol, or
a pharmaceutically acceptable salt thereof.

2. The alcohol peptide derivative of claim 1 wherein A₂ is Phe, β-(2- or 3-thienyl)alanine, or Tyr.

3. The alcohol peptide derivative of claim 1 or 2 wherein A₃ is Glu.

4. The alcohol peptide derivative of any one of claims 1 to 3 wherein A₄ is Glu, Ala, or Pro.

5. The alcohol peptide derivative of any one of claims 1 to 4 wherein A₅ is Ile.

6. The alcohol peptide derivative of any one of claims 1 to 5 wherein A₆ is Pro.

7. The alcohol peptide derivative of any one of claims 1 to 6 wherein A₇ is Glu or Ala.

8. The alcohol peptide derivative of any one of claims 1 to 7 wherein A₈ is Glu or Asp.

9. The alcohol peptide derivative of any one of claims 1 to 8 wherein A₉ is Tyr-Leu, Ala-Tyr, or Ala-Cha.

10. The alcohol peptide derivative of any one of claims 1 to 9 wherein A₁₀-ol is Gln-ol, Asp-ol, Pro-ol, Asn-ol, Asp-Glu-ol, Glu-ol, Ala-ol, D-Lys-ol, Lys-ol, D-Asp-ol, D-Glu-ol or Orn-ol.

11. The alcohol peptide derivative of any one of claims 1 to 10 wherein X is H, acetyl, or succinyl.

12. The alcohol peptide derivative of any one of claims 1 to 11 wherein
A₁ is Ser-His-Asn-Asp-Gly-Asp,
Asn-Asp-Gly-Asp,
Asp-Gly-Asp,
Gly-Asp,
Asp, or
a bond.

13. The alcohol peptide derivative of any one of claims 1 to 11 wherein A₁ is -Thr-Pro-Lys-Pro-Gln-Ser-His-Asn-Asp-Gly-Asp-.

14. The alcohol peptide derivative of any one of claims 1 to 11 wherein A₁ is -Thr-Pro-Asn-Pro-Glu-Ser-His-Asn-Asn-Gly-Asp-.

15. The alcohol peptide derivative of claim 1 which is H-Gly-Asp-Phe-Glu-Glu-Ile-Pro-Glu-Glu-Tyr-Leu-Gln-ol.

16. The alcohol peptide derivative of claim 1 which is Suc-Phe-Glu-Glu-Ile-Pro-Glu-Glu-Tyr-Leu-Gln-ol.

17. The alcohol peptide derivative of claim 1 which is H-Gly-Asp-Phe-Glu-Pro-Ile-Pro-Glu-Asp-Ala-Tyr-Asp-Glu-ol.

18. The alcohol peptide derivative of claim 1 which is Suc-Phe-Glu-Pro-Ile-Pro-Glu-Glu-Tyr-Leu-Pro-ol.

19. The alcohol peptide derivative of claim 1 which is Suc-Phe-Glu-Glu-Ile-Pro-Glu-Glu-Tyr-Leu-Gln-ol.

20. The alcohol peptide derivative of claim 1 which is Suc-Phe-Glu-Pro-Ile-Pro-Glu-Glu-Tyr-Leu-Gln-ol.

21. The alcohol peptide derivative of claim 1 which is Suc-Tyr-Glu-Pro-Ile-Pro-Glu-Glu-Ala-Phe-D-Lys-ol.

22. The alcohol peptide derivative of claim 1 which is Suc-Tyr-Glu-Pro-Ile-Pro-Glu-Glu-Ala-Tyr-Lys-ol.

23. The alcohol peptide derivative of claim 1 which is Suc-Tyr-Glu-Pro-Ile-Pro-Glu-Glu-Ala-Cha-Lys-ol.

24. The alcohol peptide derivative of claim 1 which is Suc-Tyr-Glu-Pro-Ile-Pro-Glu-Glu-Ala-Phe-Glu-ol.

25. The alcohol peptide derivative of claim 1 which is Suc-Tyr-Glu-Pro-Ile-Pro-Glu-Glu-Ala-Phe-Gln-ol.

26. The alcohol peptide derivative of claim 1 which is Suc-Tyr-Glu-Pro-Ile-Pro-Glu-Glu-Ala-Cha-Asp-ol.

27. A process for the preparation of an alcohol peptide derivative according to any one of claims 1 to 26 which comprises
(a) binding a suitably protected amino acid corresponding to the A₉ amino acid to an activated resin support
(b) subsequently binding the other alpha amino protected amino acids in order from the carboxy terminus to the amino terminus, A₈ through A₁, to the amino terminus of the growing peptidic chain which has meanwhile been exposed by removing its amino protecting group; and
(c) finally treating the resin bound peptide with about 2 equivalents of the carboxy terminal amino alcohol corresponding to A₁₀-ol and about 1 equivalent of acetic acid in dichloromethan to release the peptide from the resin and concomitantly bind the carboxy terminal peptide alcohol to the peptide thus resulting in the desired peptide alcohol derivative.

28. A peptide derivative of any one of claims 1 to 26 or a mixture thereof for use as a pharmaceutically active substance.

29. A peptide derivative of any one of claims 1 to 26 or a mixture thereof for use as an anticoagulant.

30. A pharmaceutical composition containing an anticoagulant effective amount of a peptide derivative of any one of claims 1 to 26 or of a mixture thereof and optionally a pharmaceutically acceptable carrier and/or diluent.

## Claims (Claims for the following Contracting State(s): ES)

1. A process for preparing a peptide alcohol derivative of the formula
X-A₁-A₂-A₃-A₄-A₅-A₆-A₇-A₈-A₉-A₁₀-ol
wherein
X is a hydrogen, one or two alkyl groups of from 1 to 6 carbon atoms, or one or two acyl groups of from 2 to 10 carbon atoms;
A₁ is a bond or is a peptide fragment containing from one to eleven residues of any amino acid;
A₂ is Phe, SubPhe, β-(2- and 3-thienyl)alanine, β-(2- and 3-furanyl)alanine, β-(2-, 3-, and 4-pyridyl) alanine, β-(benzothienyl-2- and 3-yl)alanine, β-(1- and 2-naphthyl)alanine, Tyr, or Trp;
A₃ is Glu or Asp;
A₄ is any amino acid;
A₅ is Ile, Val, Leu, Nle, or Phe;
A₆ is Pro, Hyp, 3,4-dehydroPro, thiazolidine-4-carboxylate, Sar, NMePgl, or D-Ala;
A₇ is any amino acid;
A₈ is any amino acid;
A₉ is a lipophilic amino acid selected from Tyr, Trp, Phe, Leu, Nle, Ile, Val, Cha and Pro or is a dipeptide containing at least one of these lipophilic amino acids;
A₁₀-ol is a reduced peptide fragment containing from zero to five residues of any amino acid wherein the carbon terminal amino acid is reduced to its alcohol derivative, or ol
which comprises
(a) binding a suitably protected amino acid corresponding to the A₉ amino acid to an activated resin support
(b) subsequently binding the other alpha amino protected amino acids in order from the carboxy terminus to the amino terminus, A₈ through A₁, to the amino terminus of the growing peptidic chain which has meanwhile been exposed by removing its amino protecting group; and
(c) finally treating the resin bound peptide with about 2 equivalents of the carboxy terminal amino alcohol corresponding to A₁₀-ol and about 1 equivalent of acetic acid in dichloromethane to release the peptide from the resin and concomitantly bind the carboxy terminal peptide alcohol to the peptide thus resulting in the desired peptide alcohol derivative.

2. The process according to claim 1 wherein A₂ is Phe, β-(2- or 3-thienyl)alanine, or Tyr.

3. The process according to claim 1 or 2 wherein A₃ is Glu.

4. The process according to any one of claims 1 to 3 wherein A₄ is Glu, Ala, or Pro.

5. The process according to any one of claims 1 to 4 wherein A₅ is Ile.

6. The process according to any one of claims 1 to 5 wherein A₆ is Pro.

7. The process according to any one of claims 1 to 6 wherein A₇ is Glu or Ala.

8. The process according to any one of claims 1 to 7 wherein A₈ is Glu or Asp.

9. The process according to any one of claims 1 to 8 wherein A₉ is Tyr-Leu, Ala-Tyr, or Ala-Cha.

10. The process according to any one of claims 1 to 9 wherein A₁₀-ol is Gln-ol, Asp-ol, Pro-ol, Asn-ol, Asp-Glu-ol, Glu-ol, Ala-ol, D-Lys-ol, Lys-ol, D-Asp-ol, D-Glu-ol or Orn-ol.

11. The process according to any one of claims 1 to 10 wherein X is H, acetyl, or succinyl.

12. The process according to any one of claims 1 to 11 wherein
A₁ is Ser-His-Asn-Asp-Gly-Asp,
Asn-Asp-Gly-Asp,
Asp-Gly-Asp,
Gly-Asp,
Asp, or
a bond.

13. The process according to any one of claims 1 to 11 wherein A₁ is -Thr-Pro-Lys-Pro-Gln-Ser-His-Asn-Asp-Gly-Asp-.

14. The process according to any one of claims 1 to 11 wherein A₁ is -Thr-Pro-Asn-Pro-Glu-Ser-His-Asn-Asn-Gly-Asp-.

15. The process according to claim 1 wherein the peptide alcohol derivative is H-Gly-Asp-Phe-Glu-Glu-Ile-Pro-Glu-Glu-Tyr-Leu-Gln-ol.

16. The process according to claim 1 wherein the peptide alcohol derivative is Suc-Phe-Glu-Glu-Ile-Pro-Glu-Glu-Tyr-Leu-Gln-ol.

17. The process according to claim 1 wherein the peptide alcohol derivative is H-Gly-Asp-Phe-Glu-Pro-Ile-Pro-Glu-Asp-Ala-Tyr-Asp-Glu-ol.

18. The process according to claim 1 wherein the peptide alcohol derivative is Suc-Phe-Glu-Pro-Ile-Pro-Glu-Glu-Tyr-Leu-Pro-ol.

19. The process according to claim 1 wherein the peptide alcohol derivative is Suc-Phe-Glu-Glu-Ile-Pro-Glu-Glu-Tyr-Leu-Gln-ol.

20. The process according to claim 1 wherein the peptide alcohol derivative is Suc-Phe-Glu-Pro-Ile-Pro-Glu-Glu-Tyr-Leu-Gln-ol.

21. The process according to claim 1 wherein the peptide alcohol derivative is Suc-Tyr-Glu-Pro-Ile-Pro-Glu-Glu-Ala-Phe-D-Lys-ol.

22. The process according to claim 1 wherein the peptide alcohol derivative is Suc-Tyr-Glu-Pro-Ile-Pro-Glu-Glu-Ala-Tyr-Lys-ol.

23. The process according to claim 1 wherein the peptide alcohol derivative is Suc-Tyr-Glu-Pro-Ile-Pro-Glu-Glu-Ala-Cha-Lys-ol.

24. The process according to claim 1 wherein the peptide alcohol derivative is Suc-Tyr-Glu-Pro-Ile-Pro-Glu-Glu-Ala-Phe-Glu-ol.

25. The process according to claim 1 wherein the peptide alcohol derivative is Suc-Tyr-Glu-Pro-Ile-Pro-Glu-Glu-Ala-Phe-Gln-ol.

26. The process according to claim 1 wherein the peptide alcohol derivative is Suc-Tyr-Glu-Pro-Ile-Pro-Glu-Glu-Ala-Cha-Asp-ol.

27. Use of a compound obtainable according to a process of any one of claims 1 to 26 for the preparation of a pharmaceutical composition.

28. Use of a compound obtainable according to a process of any one of claims 1 to 26 for the preparation of an anticoagulant.

## Claims (Claims for the following Contracting State(s): GR)

1. An alcohol peptide derivative of the formula
X-A₁-A₂-A₃-A₄-A₅-A₆-A₇-A₈-A₉-A₁₀-ol
wherein
X is a hydrogen, one or two alkyl groups of from 1 to 6 carbon atoms, or one or two acyl groups of from 2 to 10 carbon atoms;
A₁ is a bond or is a peptide fragment containing from one to eleven residues of any amino acid;
A₂ is Phe, SubPhe, β-(2- and 3-thienyl)alanine, β-(2- and 3-furanyl)alanine, β-(2-, 3-, and 4-pyridyl) alanine, β-(benzothienyl-2- and 3-yl)alanine, β-(1- and 2-naphthyl)alanine, Tyr, or Trp;
A₃ is Glu or Asp;
A₄ is any amino acid;
A₅ is Ile, Val, Leu, Nle, or Phe;
A₆ is Pro, Hyp, 3,4-dehydroPro, thiazolidine-4-carboxylate, Sar, NMePgl, or D-Ala;
A₇ is any amino acid;
A₈ is any amino acid;
A₉ is a lipophilic amino acid selected from Tyr, Trp, Phe, Leu, Nle, Ile, Val, Cha and Pro or is a dipeptide containing at least one of these lipophilic amino acids;
A₁₀-ol is a reduced peptide fragment containing from zero to five residues of any amino acid wherein the carbon terminal amino acid is reduced to its alcohol derivative; or -ol, or
a pharmaceutically acceptable salt thereof.

2. The alcohol peptide derivative of claim 1 wherein A₂ is Phe, β-(2- or 3-thienyl)alanine, or Tyr.

3. The alcohol peptide derivative of claim 1 or 2 wherein A₃ is Glu.

4. The alcohol peptide derivative of any one of claims 1 to 3 wherein A₄ is Glu, Ala, or Pro.

5. The alcohol peptide derivative of any one of claims 1 to 4 wherein A₅ is Ile.

6. The alcohol peptide derivative of any one of claims 1 to 5 wherein A₆ is Pro.

7. The alcohol peptide derivative of any one of claims 1 to 6 wherein A₇ is Glu or Ala.

8. The alcohol peptide derivative of any one of claims 1 to 7 wherein A₈ is Glu or Asp.

9. The alcohol peptide derivative of any one of claims 1 to 8 wherein A₉ is Tyr-Leu, Ala-Tyr, or Ala-Cha.

10. The alcohol peptide derivative of any one of claims 1 to 9 wherein A₁₀-ol is Gln-ol, Asp-ol, Pro-ol, Asn-ol, Asp-Glu-ol, Glu-ol, Ala-ol, D-Lys-ol, Lys-ol, D-Asp-ol, D-Glu-ol or Orn-ol.

11. The alcohol peptide derivative of any one of claims 1 to 10 wherein X is H, acetyl, or succinyl.

12. The alcohol peptide derivative of any one of claims 1 to 11 wherein
A₁ is Ser-His-Asn-Asp-Gly-Asp,
Asn-Asp-Gly-Asp,
Asp-Gly-Asp,
Gly-Asp,
Asp, or
a bond.

13. The alcohol peptide derivative of any one of claims 1 to 11 wherein A₁ is -Thr-Pro-Lys-Pro-Gln-Ser-His-Asn-Asp-Gly-Asp-.

14. The alcohol peptide derivative of any one of claims 1 to 11 wherein A₁ is -Thr-Pro-Asn-Pro-Glu-Ser-His-Asn-Asn-Gly-Asp-.

15. The alcohol peptide derivative of claim 1 which is H-Gly-Asp-Phe-Glu-Glu-Ile-Pro-Glu-Glu-Tyr-Leu-Gln-ol.

16. The alcohol peptide derivative of claim 1 which is Suc-Phe-Glu-Glu-Ile-Pro-Glu-Glu-Tyr-Leu-Gln-ol.

17. The alcohol peptide derivative of claim 1 which is H-Gly-Asp-Phe-Glu-Pro-Ile-Pro-Glu-Asp-Ala-Tyr-Asp-Glu-ol.

18. The alcohol peptide derivative of claim 1 which is Suc-Phe-Glu-Pro-Ile-Pro-Glu-Glu-Tyr-Leu-Pro-ol.

19. The alcohol peptide derivative of claim 1 which is Suc-Phe-Glu-Glu-Ile-Pro-Glu-Glu-Tyr-Leu-Gln-ol.

20. The alcohol peptide derivative of claim 1 which is Suc-Phe-Glu-Pro-Ile-Pro-Glu-Glu-Tyr-Leu-Gln-ol.

21. The alcohol peptide derivative of claim 1 which is Suc-Tyr-Glu-Pro-Ile-Pro-Glu-Glu-Ala-Phe-D-Lys-ol.

22. The alcohol peptide derivative of claim 1 which is Suc-Tyr-Glu-Pro-Ile-Pro-Glu-Glu-Ala-Tyr-Lys-ol.

23. The alcohol peptide derivative of claim 1 which is Suc-Tyr-Glu-Pro-Ile-Pro-Glu-Glu-Ala-Cha-Lys-ol.

24. The alcohol peptide derivative of claim 1 which is Suc-Tyr-Glu-Pro-Ile-Pro-Glu-Glu-Ala-Phe-Glu-ol.

25. The alcohol peptide derivative of claim 1 which is Suc-Tyr-Glu-Pro-Ile-Pro-Glu-Glu-Ala-Phe-Gln-ol.

26. The alcohol peptide derivative of claim 1 which is Suc-Tyr-Glu-Pro-Ile-Pro-Glu-Glu-Ala-Cha-Asp-ol.

27. A process for the preparation of an alcohol peptide derivative according to any one of claims 1 to 26 which comprises
(a) binding a suitably protected amino acid corresponding to the A₉ amino acid to an activated resin support
(b) subsequently binding the other alpha amino protected amino acids in order from the carboxy terminus to the amino terminus, A₈ through A₁, to the amino terminus of the growing peptidic chain which has meanwhile been exposed by removing its amino protecting group; and
(c) finally treating the resin bound peptide with about 2 equivalents of the carboxy terminal amino alcohol corresponding to A₁₀-ol and about 1 equivalent of acetic acid in dichloromethan to release the peptide from the resin and concomitantly bind the carboxy terminal peptide alcohol to the peptide thus resulting in the desired peptide alcohol derivative.

28. A peptide derivative of any one of claims 1 to 26 or a mixture thereof for use as a pharmaceutically active substance.

29. A peptide derivative of any one of claims 1 to 26 or a mixture thereof for use as an anticoagulant.

## Patentansprüche (Patentansprüche für folgende(n) Vertragsstaat(en): AT, BE, CH, DE, FR, GB, IT, LI, LU, NL, SE)

1. Alkohol-Peptidderivat der Formel
X-A₁-A₂-A₃-A₄-A₅-A₆-A₇-A₈-A₉-A₁₀-ol
wobei
X ein Wasserstoffatom, ein oder zwei Alkylrest(e) mit 1 bis 6 Kohlenstoffatomen oder ein oder zwei Acylrest(e) mit 2 bis 10 Kohlenstoffatomen ist;
A₁ eine Bindung ist oder ein einen bis elf Rest(e) einer beliebigen Aminosäure enthaltendes Peptidfragment ist;
A₂ Phe, SubPhe, β-(2- und 3-Thienyl)-alanin, β-(2- und 3-Furanyl)-alanin, β-(2-, 3- und 4-Pyridyl)-alanin, β-(Benzothienyl-2- und 3-yl)-alanin, β-(1- und 2-Naphthyl)-alanin, Tyr oder Trp ist;
A₃ Glu oder Asp ist;
A₄ eine beliebige Aminosäure ist;
A₅ Ile, Val, Leu, Nle oder Phe ist;
A₆ Pro, Hyp, 3,4 -DehydroPro, Thiazolidin-4-carboxylat, Sar, NMePgl oder D-Ala ist;
A₇ eine beliebige Aminosäure ist;
A₈ eine beliebige Aminosäure ist;
A₉ eine lipophile Aminosäure, ausgewählt aus Tyr, Trp, Phe, Leu, Nle, Ile, Val, Cha und Pro, oder ein Dipeptid, enthaltend mindestens eine dieser lipophilen Aminosäuren, ist;
A₁₀-ol ein reduziertes, null bis fünf Reste einer beliebigen Aminosäure enthaltendes Peptidfragment ist, wobei die Kohlenstoff-terminale Aminosäure zu ihrem Alkohol-Derivat reduziert ist, oder -ol ist; oder ein pharmazeutisch verträgliches Salz davon.

2. Alkohol-Peptidderivat nach Anspruch 1, wobei A₂ Phe, β-(2- oder 3-Thienyl)-alanin oder Tyr ist.

3. Alkohol-Peptidderivat nach Anspruch 1 oder 2, wobei A₃ Glu ist.

4. Alkohol-Peptidderivat nach einem der Ansprüche 1 bis 3, wobei A₄ Glu, Ala oder Pro ist.

5. Alkohol-Peptidderivat nach einem der Ansprüche 1 bis 4, wobei A₅ Ile ist.

6. Alkohol-Peptidderivat nach einem der Ansprüche 1 bis 5, wobei A₆ Pro ist.

7. Alkohol-Peptidderivat nach einem der Ansprüche 1 bis 6, wobei A₇ Glu oder Ala ist.

8. Alkohol-Peptidderivat nach einem der Ansprüche 1 bis 7, wobei A₈ Glu oder Asp ist.

9. Alkohol-Peptidderivat nach einem der Ansprüche 1 bis 8, wobei A₉ Tyr-Leu, Ala-Tyr oder Ala-Cha ist.

10. Alkohol-Peptidderivat nach einem der Ansprüche 1 bis 9, wobei A₁₀-ol Gln-ol, Asp-ol, Pro-ol, Asn-ol, Asp-Glu-ol, Glu-ol, Ala-ol, D-Lys-ol, Lys-ol, D-Asp-ol, D-Glu-ol oder Orn-ol ist.

11. Alkohol-Peptidderivat nach einem der Ansprüche 1 bis 10, wobei X H, eine Acetyl- oder eine Succinylgruppe ist.

12. Alkohol-Peptidderivat nach einem der Ansprüche 1 bis 11, wobei
A₁ Ser-His-Asn-Asp-Gly-Asp,
Asn-Asp-Gly-Asp,
Asp-Gly-Asp,
Gly-Asp,
Asp oder
eine Bindung ist.

13. Alkohol-Peptidderivat nach einem der Ansprüche 1 bis 11, wobei A₁ -Thr-Pro-Lys-Pro-Gln-Ser-His-Asn-Asp-Gly-Asp- ist.

14. Alkohol-Peptidderivat nach einem der Ansprüche 1 bis 11, wobei A₁ -Thr-Pro-Asn-Pro-Glu-Ser-His-Asn-Asn-Gly-Asp- ist.

15. Alkohol-Peptidderivat nach Anspruch 1, das H-Gly-Asp-Phe-Glu-Glu-Ile-Pro-Glu-Glu-Tyr-Leu-Gln-ol ist.

16. Alkohol-Peptidderivat nach Anspruch 1, das Suc-Phe-Glu-Glu-Ile-Pro-Glu-Glu-Tyr-Leu-Gln-ol ist.

17. Alkohol-Peptidderivat nach Anspruch 1, das H-Gly-Asp-Phe-Glu-Pro-Ile-Pro-Glu-Asp-Ala-Tyr-Asp-Glu-ol ist.

18. Alkohol-Peptidderivat nach Anspruch 1, das Suc-Phe-Glu-Pro-Ile-Pro-Glu-Glu-Tyr-Leu-Pro-ol ist.

19. Alkohol-Peptidderivat nach Anspruch 1, das Suc-Phe-Glu-Glu-Ile-Pro-Glu-Glu-Tyr-Leu-Gln-ol ist.

20. Alkohol-Peptidderivat nach Anspruch 1, das Suc-Phe-Glu-Pro-Ile-Pro-Glu-Glu-Tyr-Leu-Gln-ol ist.

21. Alkohol-Peptidderivat nach Anspruch 1, das Suc-Tyr-Glu-Pro-Ile-Pro-Glu-Glu-Ala-Phe-D-Lys-ol ist.

22. Alkohol-Peptidderivat nach Anspruch 1, das Suc-Tyr-Glu-Pro-Ile-Pro-Glu-Glu-Ala-Tyr-Lys-ol ist.

23. Alkohol-Peptidderivat nach Anspruch 1, das Suc-Tyr-Glu-Pro-Ile-Pro-Glu-Glu-Ala-Cha-Lys-ol ist.

24. Alkohol-Peptidderivat nach Anspruch 1, das Suc-Tyr-Glu-Pro-Ile-Pro-Glu-Glu-Ala-Phe-Glu-ol ist.

25. Alkohol-Peptidderivat nach Anspruch 1, das Suc-Tyr-Glu-Pro-Ile-Pro-Glu-Glu-Ala-Phe-Gln-ol ist.

26. Alkohol-Peptidderivat nach Anspruch 1, das Suc-Tyr-Glu-Pro-Ile-Pro-Glu-Glu-Ala-Cha-Asp-ol ist.

27. Verfahren zur Herstellung eines Alkohol-Peptidderivates nach einem der Ansprüche 1 bis 26, welches umfaßt
(a) Bindung einer geeignet geschützten, der A₉-Aminosäure entsprechenden Aminosäure an einen aktivierten Harzträger;
(b) nachfolgende Bindung der anderen alpha-Amino-geschützten Aminosäuren in der Abfolge vom Carboxy-Ende bis zum Amino-Ende, von A₈ durchgehend bis A₁, an das Amino-Ende der wachsenden Peptidkette, das inzwischen durch Entfernung seiner Amino-Schutzgruppe freigelegt worden ist; und
(c) zuletzt Behandlung des an das Harz gebundenen Peptides mit etwa 2 Äquivalenten des A₁₀-ol entsprechenden Carboxyterminalen Aminoalkohols und etwa 1 Äquivalent Essigsäure in Dichlormethan, um das Peptid vom Harz freizusetzen und gleichzeitig den Carboxy-terminalen Peptid-Alkohol an das Peptid zu binden, wodurch sich das gewünschte Peptid-Alkoholderivat ergibt.

28. Peptidderivat nach einem der Ansprüche 1 bis 26 oder ein Gemisch davon zur Verwendung als pharmazeutischer Wirkstoff.

29. Peptidderivat nach einem der Ansprüche 1 bis 26 oder ein Gemisch davon zur Verwendung als Antikoagulans.

30. Arzneimittel, enthaltend eine als Antikoagulans wirksame Menge eines Peptidderivates nach einem der Ansprüche 1 bis 26 oder eines Gemisches davon und gegebenenfalls ein pharmazeutisch verträgliches Trägermittel und/oder Verdünnungsmittel.

## Patentansprüche (Patentansprüche für folgende(n) Vertragsstaat(en): ES)

1. Verfahren zur Herstellung eines Peptid-Alkoholderivates der Formel
X-A₁-A₂-A₃-A₄-A₅-A₆-A₇-A₈-A₉-A₁₀-ol
wobei
X ein Wasserstoffatom, ein oder zwei Alkylrest(e) mit 1 bis 6 Kohlenstoffatomen oder ein bis zwei Acylrest(e) mit 2 bis 10 Kohlenstoffatomen ist;
A₁ eine Bindung ist oder ein einen bis elf Rest(e) einer beliebigen Aminosäure enthaltendes Peptidfragment ist;
A₂ Phe, SubPhe, β-(2- und 3-Thienyl)-alanin, β-(2- und 3-Furanyl)-alanin, β-(2-, 3- und 4-Pyridyl)-alanin, β-(Benzothienyl-2- und 3-yl)-alanin, β-( 1- und 2-Naphthyl)-alanin, Tyr oder Trp ist;
A₃ Glu oder Asp ist;
A₄ eine beliebige Aminosäure ist;
A₅ Ile, Val, Leu, Nle oder Phe ist;
A₆ Pro, Hyp, 3,4 -DehydroPro, Thiazolidin-4-carboxylat, Sar, NMePgl oder D-Ala ist;
A₇ eine beliebige Aminosäure ist;
A₈ eine beliebige Aminosäure ist;
A₉ eine lipophile Aminosäure, ausgewählt aus Tyr, Trp, Phe, Leu, Nle, Ile, Val, Cha und Pro, oder ein Dipeptid, enthaltend mindestens eine dieser lipophilen Aminosäuren, ist;
A₁₀-ol ein reduziertes, null bis fünf Reste einer beliebigen Aminosäure enthaltendes Peptidfragment ist, wobei die Kohlenstoff-terminale Aminosäure zu ihrem Alkohol-Derivat reduziert ist, oder -ol ist,
welches umfaßt
(a) Bindung einer geeignet geschützten, der A₉-Aminosäure entsprechenden Aminosäure an einen aktivierten Harzträger;
(b) nachfolgende Bindung der anderen alpha-Amino-geschützten Aminosäuren in der Abfolge vom Carboxy-Ende bis zum Amino-Ende, von A₈ durchgehend bis A₁, an das Amino-Ende der wachsenden Peptidkette, das inzwischen durch Entfernung seiner Amino-Schutzgruppe freigelegt worden ist; und
(c) zuletzt Behandlung des an das Harz gebundenen Peptides mit etwa 2 Äquivalenten des A₁₀-ol entsprechenden Carboxy-terminalen Aminoalkohols und etwa 1 Äquivalent Essigsäure in Dichoromethan, um das Peptid aus dem Harz freizusetzen und gleichzeitig den Carboxy-terminalen Peptid-Alkohol an das Peptid zu binden, wodurch sich das gewünschte Peptid-Alkoholderivat ergibt.

2. Verfahren nach Anspruch 1, wobei A₂ Phe, β-(2- oder 3-Thienyl)-alanin oder Tyr ist.

3. Verfahren nach Anspruch 1 oder 2, wobei A₃ Glu ist.

4. Verfahren nach einem der Ansprüche 1 bis 3, wobei A₄ Glu, Ala oder Pro ist.

5. Verfahren nach einem der Ansprüche 1 bis 4, wobei A₅ Ile ist.

6. Verfahren nach einem der Ansprüche 1 bis 5, wobei A₆ Pro ist.

7. Verfahren nach einem der Ansprüche 1 bis 6, wobei A₇ Glu oder Ala ist.

8. Verfahren nach einem der Ansprüche 1 bis 7, wobei A₈ Glu oder Asp ist.

9. Verfahren nach einem der Ansprüche 1 bis 8, wobei A₉ Tyr-Leu Ala-Tyr oder Ala-Cha ist.

10. Verfahren nach einem der Ansprüche 1 bis 9, wobei A₁₀-ol Gln-ol, Asp-ol, Pro-ol, Asn-ol, Asp-Glu-ol, Glu-ol, Ala-ol, D-Lys-ol, Lys-ol, D-Asp-ol, D-Glu-ol oder Orn-ol ist.

11. Verfahren nach einem der Ansprüche 1 bis 10, wobei X H, eine Acetyl- oder eine Succinylgruppe ist.

12. Verfahren nach einem der Ansprüche 1 bis 11, wobei
A₁ Ser-His-Asn-Asp-Gly-Asp,
Asn-Asp-Gly-Asp,
Asp-Gly-Asp,
Gly-Asp,
Asp oder
eine Bindung ist.

13. Verfahren nach einem der Ansprüche 1 bis 11, wobei A₁ -Thr-Pro-Lys-Pro-Gln-Ser-His-Asn-Asp-Gly-Asp- ist.

14. Verfahren nach einem der Ansprüche 1 bis 11, wobei A₁ -Thr-Pro-Asn-Pro-Glu-Ser-His-Asn-Asn-Gly-Asp- ist.

15. Verfahren nach Anspruch 1, wobei das Peptid-Alkoholderivat H-Gly-Asp-Phe-Glu-Glu-Ile-Pro-Glu-Glu-Tyr-Leu-Gln-ol ist.

16. Verfahren nach Anspruch 1, wobei das Peptid-Alkoholderivat Suc-Phe-Glu-Glu-Ile-Pro-Glu-Glu-Tyr-Leu-Gln-ol ist.

17. Verfahren nach Anspruch 1, wobei das Peptid-Alkoholderivat H-Gly-Asp-Phe-Glu-Pro-Ile-Pro-Glu-Asp-Ala-Tyr-Asp-Glu-ol ist.

18. Verfahren nach Anspruch 1, wobei das Peptid-Alkoholderivat Suc-Phe-Glu-Pro-Ile-Pro-Glu-Glu-Tyr-Leu-Pro-ol ist.

19. Verfahren nach Anspruch 1, wobei das Peptid-Alkoholderivat Suc-Phe-Glu-Glu-Ile-Pro-Glu-Glu-Tyr-Leu-Gln-ol ist.

20. Verfahren nach Anspruch 1, wobei das Peptid-Alkoholderivat Suc-Phe-Glu-Pro-Ile-Pro-Glu-Glu-Tyr-Leu-Gln-ol ist.

21. Verfahren nach Anspruch 1, wobei das Peptid-Alkoholderivat Suc-Tyr-Glu-Pro-Ile-Pro-Glu-Glu-Ala-Phe-D-Lys-ol ist.

22. Verfahren nach Anspruch 1, wobei das Peptid-Alkoholderivat Suc-Tyr-Glu-Pro-Ile-Pro-Glu-Glu-Ala-Tyr-Lys-ol ist.

23. Verfahren nach Anspruch 1, wobei das Peptid-Alkoholderivat Suc-Tyr-Glu-Pro-Ile-Pro-Glu-Glu-Ala-Cha-Lys-ol ist.

24. Verfahren nach Anspruch 1, wobei das Peptid-Alkoholderivat Suc-Tyr-Glu-Pro-Ile-Pro-Glu-Glu-Ala-Phe-Glu-ol ist.

25. Verfahren nach Anspruch 1, wobei das Peptid-Alkoholderivat Suc-Tyr-Glu-Pro-Ile-Pro-Glu-Glu-Ala-Phe-Gln-ol ist.

26. Verfahren nach Anspruch 1, wobei das Peptid-Alkoholderivat Suc-Tyr-Glu-Pro-Ile-Pro-Glu-Glu-Ala-Cha-Asp-ol ist.

27. Verwendung einer Verbindung, erhältlich gemäß einem Verfahren nach einem der Ansprüche 1 bis 26, zur Herstellung eines Arzneimittels.

28. Verwendung einer Verbindung, erhältlich gemäß einem Verfahren nach einem der Ansprüche 1 bis 26, zur Herstellung eines Antikoagulans.

## Patentansprüche (Patentansprüche für folgende(n) Vertragsstaat(en): GR)

1. Alkohol-Peptidderivat der Formel
X-A₁-A₂-A₃-A₄-A₅-A₆-A₇-A₈-A₉-A₁₀-ol
wobei
X ein Wasserstoffatom, ein oder zwei Alkylrest(e) mit 1 bis 6 Kohlenstoffatomen oder ein oder zwei Acylrest(e) mit 2 bis 10 Kohlenstoffatomen ist;
A₁ eine Bindung ist oder ein einen bis elf Rest(e) einer beliebigen Aminosäure enthaltendes Peptidfragment ist;
A₂ Phe, SubPhe, β-(2- und 3-Thienyl)-alanin, β-(2- und 3-Furanyl)-alanin, β-(2-, 3- und 4-Pyridyl)-alanin, β-(Benzothienyl-2- und 3-yl)-alanin, β-( 1- und 2-Naphthyl)-alanin, Tyr oder Trp ist;
A₃ Glu oder Asp ist;
A₄ eine beliebige Aminosäure ist;
A₅ Ile, Val, Leu, Nle oder Phe ist;
A₆ Pro, Hyp, 3,4 -DehydroPro, Thiazolidin-4-carboxylat, Sar, NMePgl oder D-Ala ist;
A₇ eine beliebige Aminosäure ist;
A₈ eine beliebige Aminosäure ist;
A₉ eine lipophile Aminosäure, ausgewählt aus Tyr, Trp, Phe, Leu, Nle, Ile, Val, Cha und Pro, oder ein Dipeptid, enthaltend mindestens eine dieser lipophilen Aminosäuren, ist;
A₁₀-ol ein reduziertes, null bis fünf Reste einer beliebigen Aminosäure enthaltendes Peptidfragment ist, wobei die Kohlenstoff-terminale Aminosäure zu ihrem Alkohol-Derivat reduziert ist, oder -ol ist; oder ein pharmazeutisch verträgliches Salz davon.

2. Alkohol-Peptidderivat nach Anspruch 1, wobei A₂ Phe, β-(2- oder 3-Thienyl)-alanin oder Tyr ist.

3. Alkohol-Peptidderivat nach Anspruch 1 oder 2, wobei A₃ Glu ist.

4. Alkohol-Peptidderivat nach einem der Ansprüche 1 bis 3, wobei A₄ Glu, Ala oder Pro ist.

5. Alkohol-Peptidderivat nach einem der Ansprüche 1 bis 4, wobei A₅ Ile ist.

6. Alkohol-Peptidderivat nach einem der Ansprüche 1 bis 5, wobei A₆ Pro ist.

7. Alkohol-Peptidderivat nach einem der Ansprüche 1 bis 6, wobei A₇ Glu oder Ala ist.

8. Alkohol-Peptidderivat nach einem der Ansprüche 1 bis 7, wobei A₈ Glu oder Asp ist.

9. Alkohol-Peptidderivat nach einem der Ansprüche 1 bis 8, wobei A₉ Tyr-Leu, Ala-Tyr oder Ala-Cha ist.

10. Alkohol-Peptidderivat nach einem der Ansprüche 1 bis 9, wobei A₁₀-ol Gln-ol, Asp-ol, Pro-ol, Asn-ol, Asp-Glu-ol, Glu-ol, Ala-ol, D-Lys-ol, Lys-ol, D-Asp-ol, D-Glu-ol oder Orn-ol ist.

11. Alkohol-Peptidderivat nach einem der Ansprüche 1 bis 10, wobei X H, eine Acetyl- oder eine Succinylgruppe ist.

12. Alkohol-Peptidderivat nach einem der Ansprüche 1 bis 11, wobei
A₁ Ser-His-Asn-Asp-Gly-Asp,
Asn-Asp-Gly-Asp,
Asp-Gly-Asp,
Gly-Asp,
Asp oder
eine Bindung ist.

13. Alkohol-Peptidderivat nach einem der Ansprüche 1 bis 11, wobei A₁ -Thr-Pro-Lys-Pro-Gln-Ser-His-Asn-Asp-Gly-Asp- ist.

14. Alkohol-Peptidderivat nach einem der Ansprüche 1 bis 11, wobei A₁ -Thr-Pro-Asn-Pro-Glu-Ser-His-Asn-Asn-Gly-Asp- ist.

15. Alkohol-Peptidderivat nach Anspruch 1, das H-Gly-Asp-Phe-Glu-Glu-Ile-Pro-Glu-Glu-Tyr-Leu-Gln-ol ist.

16. Alkohol-Peptidderivat nach Anspruch 1, das Suc-Phe-Glu-Glu-Ile-Pro-Glu-Glu-Tyr-Leu-Gln-ol ist.

17. Alkohol-Peptidderivat nach Anspruch 1, das H-Gly-Asp-Phe-Glu-Pro-Ile-Pro-Glu-Asp-Ala-Tyr-Asp-Glu-ol ist.

18. Alkohol-Peptidderivat nach Anspruch 1, das Suc-Phe-Glu-Pro-Ile-Pro-Glu-Glu-Tyr-Leu-Pro-ol ist.

19. Alkohol-Peptidderivat nach Anspruch 1, das Suc-Phe-Glu-Glu-Ile-Pro-Glu-Glu-Tyr-Leu-Gln-ol ist.

20. Alkohol-Peptidderivat nach Anspruch 1, das Suc-Phe-Glu-Pro-Ile-Pro-Glu-Glu-Tyr-Leu-Gln-ol ist.

21. Alkohol-Peptidderivat nach Anspruch 1, das Suc-Tyr-Glu-Pro-Ile-Pro-Glu-Glu-Ala-Phe-D-Lys-ol ist.

22. Alkohol-Peptidderivat nach Anspruch 1, das Suc-Tyr-GIu-Pro-Ile-Pro-Glu-Glu-Ala-Tyr-Lys-ol ist.

23. Alkohol-Peptidderivat nach Anspruch 1, das Suc-Tyr-Glu-Pro-Ile-Pro-Glu-Glu-Ala-Cha-Lys-ol ist.

24. Alkohol-Peptidderivat nach Anspruch 1, das Suc-Tyr-Glu-Pro-Ile-Pro-Glu-Glu-Ala-Phe-Glu-ol ist.

25. Alkohol-Peptidderivat nach Anspruch 1, das Suc-Tyr-Glu-Pro-Ile-Pro-Glu-Glu-Ala-Phe-Gln-ol ist.

26. Alkohol-Peptidderivat nach Anspruch 1, das Suc-Tyr-Glu-Pro-Ile-Pro-Glu-Glu-Ala-Cha-Asp-ol ist.

27. Verfahren zur Herstellung eines Alkohol-Peptidderivates nach einem der Ansprüche 1 bis 26, welches umfaßt
(a) Bindung einer geeignet geschützten, der A₉-Aminosäure entsprechenden Aminosäure an einen aktivierten Harzträger;
(b) nachfolgende Bindung der anderen alpha-Amino-geschützten Aminosäuren in der Abfolge vom Carboxy-Ende bis zum Amino-Ende, von A₈ durchgehend bis A₁, an das Amino-Ende der wachsenden Peptidkette, das inzwischen durch Entfernung seiner Amino-Schutzgruppe freigelegt worden ist; und
(c) zuletzt Behandlung des an das Harz gebundenen Peptides mit etwa 2 Äquivalenten des A₁₀-ol entsprechenden Carboxyterminalen Aminoalkohols und etwa 1 Äquivalent Essigsäure in Dichlormethan, um das Peptid vom Harz freizusetzen und gleichzeitig den Carboxy-terminalen Peptid-Alkohol an das Peptid zu binden, wodurch sich das gewünschte Peptid-Alkoholderivat ergibt.

28. Peptidderivat nach einem der Ansprüche 1 bis 26 oder ein Gemisch davon zur Verwendung als pharmazeutischer Wirkstoff.

29. Peptidderivat nach einem der Ansprüche 1 bis 26 oder ein Gemisch davon zur Verwendung als Antikoagulans.

## Revendications (Revendications pour l'(les) Etat(s) contractant(s) suivant(s): AT, BE, CH, DE, FR, GB, IT, LI, LU, NL, SE)

1. Dérivé de peptide alcool de formule
X-A₁-A₂-A₃-A₄-A₅-A₆-A₇-A₈-A₉-A₁₀-ol
dans laquelle
X est un hydrogène, un ou deux groupes alkyles ayant de 1 à 6 atomes de carbone, ou un ou deux groupes acyles ayant de 2 à 10 atomes de carbone ;
A₁ est une liaison ou un fragment peptidique contenant de 1 à 11 restes amino-acides quelconques ;
A₂ est Phe SubPhe, β-(2- et 3-thiényl)alanine, β-(2- et 3-furannyl)alanine, β-(2-, 3- et 4-pyridyl)alanine β-(benzothiényl-2- et -3-yl)alanine, β-(1- et 2-naphtyl)alanine, Tyr ou Trp ;
A₃ est Glu ou Asp ;
A₄ est un amino-acide quelconque ;
A₅ est Ile, Val, Leu, Nle ou Phe ;
A₆ est Pro, Hyp, 3,4-déshydroPro, thiazolidine-4-carboxylate, Sar, NMePgl ou D-Ala ;
A₇ est un amino-acide quelconque ;
A₈ est un amino-acide quelconque ;
A₉ est un amino-acide lipophile sélectionné parmi Tyr, Trp, Phe, Leu, Nle, Ile, Val, Cha et Pro ou est un dipeptide contenant au moins un de ces amino-acides lipophiles ;
A₁₀-ol est un fragment peptidique réduit contenant de 0 à 5 restes amino-acides quelconques, dans lequel l'amino-acide carbone-terminal est réduit en son dérivé alcool, ou ol, ou
un de ses sels pharmaceutiquement acceptables.

2. Dérivé de peptide alcool de la revendication 1, dans lequel A₂ est Phe, β-(2- ou 3-thiényl)alanine, ou Tyr.

3. Dérivé de peptide alcool de la revendication 1 ou 2, dans lequel A₃ est Glu.

4. Dérivé de peptide alcool d'une quelconque des revendications 1 à 3, dans lequel A₄ est Glu, Ala ou Pro.

5. Dérivé de peptide alcool d'une quelconque des revendications 1 à 4, dans lequel A₅ est Ile.

6. Dérivé de peptide alcool d'une quelconque des revendications 1 à 5, dans lequel A₆ est Pro.

7. Dérivé de peptide alcool d'une quelconque des revendications 1 à 6, dans lequel A₇ est Glu ou Ala.

8. Dérivé de peptide alcool d'une quelconque des revendications 1 à 7, dans lequel A₈ est Glu ou Asp.

9. Dérivé de peptide alcool d'une quelconque des revendications 1 à 8, dans lequel A₉ est Tyr-Leu, Ala-Tyr ou Ala-Cha.

10. Dérivé de peptide alcool d'une quelconque des revendications 1 à 9, dans lequel A₁₀-ol est Gln-ol, Asp-ol, Pro-ol, Asn-ol, Asp-Glu-ol, Glu-ol, Ala-ol, D-Lys-ol, Lys-ol, D-Asp-ol, D-Glu-ol ou Orn-ol.

11. Dérivé de peptide alcool d'une quelconque des revendications 1 à 10, dans lequel X est H, acétyle ou succinyle.

12. Dérivé de peptide alcool d'une quelconque des revendications 1 à 11, dans lequel A₁ est
Ser-His-Asn-Asp-Gly-Asp.
Asn-Asp-Gly-Asp,
Asp-Gly-Asp,
Gly-Asp,
Asp, ou
une liaison.

13. Dérivé de peptide alcool d'une quelconque des revendications 1 à 11, dans lequel A₁ est -Thr-Pro-Lys-Pro-Gln-Ser-His-Asn-Asp-Gly-Asp-.

14. Dérivé de peptide alcool d'une quelconque des revendications 1 à 11, dans lequel A₁ est -Thr-Pro-Asn-Pro-Glu-Ser-His-Asn-Asn-Gly-Asp-.

15. Dérivé de peptide alcool de la revendication 1, qui est H-Gly-Asp-Phe-Glu-Glu-Ile-Pro-Glu-Glu-Tyr-Leu-Gln-ol.

16. Dérivé de peptide alcool de la revendication 1, qui est Suc-Phe- Glu-Glu-Ile-Pro-Glu-Glu-Tyr-Leu-Gln-ol.

17. Dérivé de peptide alcool de la revendication 1, qui est H-Gly-Asp-Phe-Glu-Pro-Ile-Pro-GIu-Asp-Ala-Tyr-Asp-Glu-ol.

18. Dérivé de peptide alcool de la revendication 1, qui est Suc-Phe-Glu-Pro-Ile-Pro-Glu-Glu-Tyr-Leu-Pro-ol.

19. Dérivé de peptide alcool de la revendication 1, qui est Suc-Phe-Glu-Glu-Ile-Pro-Glu-Glu-Tyr-Leu-Gln-ol.

20. Dérivé de peptide alcool de la revendication 1, qui est Suc-Phe-Glu-Pro-Ile-Pro-Glu-Glu-Tyr-Leu-Gln-ol.

21. Dérivé de peptide alcool de la revendication 1, qui est Suc-Tyr-Glu-Pro-Ile-Pro-Glu-Glu-Ala-Phe-D-Lys-ol.

22. Dérivé de peptide alcool de la revendication 1, qui est Suc-Tyr-Glu-Pro-Ile-Pro-Glu-Glu-Ala-Tyr-Lys-ol.

23. Dérivé de peptide alcool de la revendication 1, qui est Suc-Tyr-Glu-Pro-Ile-Pro-Glu-Glu-Ala-Cha-Lys-ol.

24. Dérivé de peptide alcool de la revendication 1, qui est Suc-Tyr-Glu-Pro-Ile-Pro-Glu-Glu-Ala-Phe-Glu-ol.

25. Dérivé de peptide alcool de la revendication 1, qui est Suc-Tyr-Glu-Pro-Ile-Pro-Glu-Glu-Ala-Phe-Gln-ol.

26. Dérivé de peptide alcool de la revendication 1, qui est Suc-Tyr-Glu-Pro-Ile-Pro-Glu-Glu-Ala-Cha-Asp-ol.

27. Procédé pour la préparation d'un dérivé de peptide alcool selon une quelconque des revendications 1 à 26 qui comprend
(a) la liaison d'un amino-acide convenablement protégé correspondant à l'amino-acide A₉ à un support en résine activé.
(b) suivie de la liaison des autres amino-acides dont le groupe alpha-amino est protégé, dans l'ordre allant de l'extrémité carboxy à l'extrémité amino, de A₈ à A₁, à l'extrémité amino de la chaîne peptidique en construction, qui a été dans l'intervalle exposée par élimination de son groupement protecteur de l'amino ; et
(c) enfin le traitement du peptide lié à la résine par environ deux équivalents de l'amino-alcool carboxy-terminal correspondant à A₁₀-ol et environ 1 équivalent d'acide acétique dans le dichlorométhane pour libérer le peptide de la résine et simultanément lier l'alcool de peptide carboxy-terminal au peptide, formant ainsi le dérivé de peptide alcool voulu.

28. Dérivé de peptide d'une quelconque des revendications 1 à 26 ou un de leurs mélanges, pour leur utilisation en tant que substance pharmaceutiquement active.

29. Dérivé de peptide d'une quelconque des revendications 1 à 26 ou un de leurs mélanges, pour leur utilisation en tant qu'anticoagulant.

30. Composition pharmaceutique contenant une quantité efficace en tant qu'anticoagulant d'un dérivé de peptide d'une quelconque des revendications 1 à 26 ou d'un de leurs mélanges et optionnellement un véhicule et/ou un diluant pharmaceutiquement acceptables.

## Revendications (Revendications pour l'(les) Etat(s) contractant(s) suivant(s): ES)

1. Procédé de préparation d'un dérivé de peptide alcool de formule :
X-A₁-A₂-A₃-A₄-A₅-A₆-A₇-A₈-A₉-A₁₀-ol
dans laquelle
X est un hydrogène, un ou deux groupes alkyles ayant de 1 à 6 atomes de carbone, ou un ou deux groupes acyles ayant de 2 à 10 atomes de carbone ;
A₁ est une liaison ou un fragment peptidique contenant de 1 à 11 restes amino-acides quelconques ;
A₂ est Phe, SubPhe, β-(2- et 3-thiényl)alanine, β-(2- et 3-furannyl)alanine, β-(2-, 3- et 4-pyridyl)alanine, β-(benzothiényl-2- et -3-yl)alanine, β-(1- et 2-naphtyl)alanine, Tyr ou Trp ;
A₃ est Glu ou Asp ;
A₄ est un amino-acide quelconque ;
A₅ est Ile, Val, Leu, Nle ou Phe ;
A₆ est Pro, Hyp, 3,4-déshydroPro, thiazolidine-4-carboxylate, Sar, NMePgl ou D-Ala ;
A₇ est un amino-acide quelconque ;
A₈ est un amino-acide quelconque ;
A₉ est un amino-acide lipophile sélectionné parmi Tyr, Trp, Phe, Leu, Nle, Ile, Val, Cha et Pro ou est un dipeptide contenant au moins un de ces amino-acides lipophiles ;
A₁₀-ol est un fragment peptidique réduit contenant de 0 à 5 restes amino-acides quelconques, dans lequel l'amino-acide carbone-terminal est réduit en son dérivé alcool, ou ol,
qui comprend
(a) la liaison d'un amino-acide convenablement protégé correspondant à l'amino-acide A₉ à un support en résine activé,
(b) suivie de la liaison des autres amino-acides dont le groupe alpha-amino est protégé, dans l'ordre allant de l'extrémité carboxy à l'extrémité amino, de A₈ à A₁, à l'extrémité amino de la chaîne peptidique en construction, qui a été dans l'intervalle exposée par élimination de son groupement protecteur de l'amino ; et
(c) enfin le traitement du peptide lié à la résine par environ 2 équivalents de l'amino-alcool carboxy-terminal correspondant à A₁₀-ol et environ 1 équivalent d'acide acétique dans le dichlorométhane pour libérer le peptide de la résine et simultanément lier l'alcool de peptide carboxy-terminal au peptide, formant ainsi le dérivé de peptide alcool voulu.

2. Procédé selon la revendication 1, dans lequel A₂ est Phe, β-(2- ou 3-thiényl)alanine, ou Tyr.

3. Procédé selon la revendication 1 ou 2, dans lequel A₃ est Glu.

4. Procédé selon l'une quelconque des revendications 1 à 3, dans lequel A₄ est Glu, Ala ou Pro.

5. Procédé selon l'une quelconque des revendications 1 à 4, dans lequel A₅ est Ile.

6. Procédé selon l'une quelconque des revendications 1 à 5, dans lequel A₆ est Pro.

7. Procédé selon l'une quelconque des revendications 1 à 6, dans lequel A₇ est Glu ou Ala.

8. Procédé selon l'une quelconque des revendications 1 à 7, dans lequel A₈ est Glu ou Asp.

9. Procédé selon l'une quelconque des revendications 1 à 8, dans lequel A₉ est Tyr-Leu, Ala-Tyr ou Ala-Cha.

10. Procédé selon l'une quelconque des revendications 1 à 9, dans lequel A₁₀-ol est Gln-ol, Asp-ol, Pro-ol, Asn-ol, Asp-Glu-ol, Glu-ol, Ala-ol, D-Lys-ol, Lys-ol, D-Asp-ol, D-Glu-ol ou Orn-ol.

11. Procédé selon l'une quelconque des revendications 1 à 10, dans lequel X est H, acétyle ou succinyle.

12. Procédé selon l'une quelconque des revendications 1 à 11, dans lequel A₁ est
Ser-His-Asn-Asp-Gly-Asp,
Asn-Asp-Gly-Asp,
Asp-Gly-Asp,
Gly-Asp,
Asp, ou
une liaison.

13. Procédé selon l'une quelconque des revendications 1 à 11, dans lequel A₁ est -Thr-Pro-Lys-Pro-Gln-Ser-His-Asn-Asp-Gly-Asp-.

14. Procédé selon l'une quelconque des revendications 1 à 11, dans lequel A₁ est -Thr-Pro-Asn-Pro-Glu-Ser-His-Asn-Asn-Gly-Asp-.

15. Procédé selon la revendication 1, dans lequel le dérivé de peptide alcool est H-Gly-Asp-Phe-Glu-Glu-Ile-Pro-Glu-Glu-Tyr-Leu-Gln-ol.

16. Procédé selon la revendication 1, dans lequel le dérivé de peptide alcool est Suc-Phe-Glu -Glu-Ile-Pro-Glu-Glu-Tyr-Leu-Gln-ol.

17. Procédé selon la revendication 1, dans lequel le dérivé de peptide alcool est H-Gly-Asp-Phe-Glu-Pro-Ile-Pro-Glu-Asp-Ala-Tyr-Asp-Glu-ol.

18. Procédé selon la revendication 1, dans lequel le dérivé de peptide alcool est Suc-Phe-Glu-Pro-Ile-Pro-Glu-Glu-Tyr-Leu-Pro-ol.

19. Procédé selon la revendication 1, dans lequel le dérivé de peptide alcool est Suc-Phe-Glu-Glu-Ile-Pro-Glu-Glu-Tyr-Leu-Gln-ol.

20. Procédé selon la revendication 1, dans lequel le dérivé de peptide alcool est Suc-Phe-Glu-Pro-Ile-Pro-Glu-Glu-Tyr-Leu-Gln-ol.

21. Procédé selon la revendication 1, dans lequel le dérivé de peptide alcool est Suc-Tyr-Glu-Pro-Ile-Pro-Glu-Glu-Ala-Phe-D-Lys-ol.

22. Procédé selon la revendication 1, dans lequel le dérivé de peptide alcool est Suc-Tyr-Glu-Pro-Ile-Pro-Glu-Glu-Ala-Tyr-Lys-ol.

23. Procédé selon la revendication 1, dans lequel le dérivé de peptide alcool est Suc-Tyr-Glu-Pro-Ile-Pro-Glu-Glu-Ala-Cha-Lys-ol.

24. Procédé selon la revendication 1, dans lequel le dérivé de peptide alcool est Suc-Tyr-Glu-Pro-Ile-Pro-Glu-Glu-Ala-Phe-Glu-ol.

25. Procédé selon la revendication 1, dans lequel le dérivé de peptide alcool est Suc-Tyr-Glu-Pro-Ile-Pro-Glu-Glu-Ala-Phe-Gln-ol.

26. Procédé selon la revendication 1, dans lequel le dérivé de peptide alcool est Suc-Tyr-Glu-Pro-Ile-Pro-Glu-Glu-Ala-Cha-Asp-ol.

27. Utilisation d'un composé que l'on peut obtenir par un procédé selon l'une quelconque des revendications 1 à 26,pour la préparation d'une composition pharmaceutique.

28. Utilisation d'un composé que l'on peut obtenir par un procédé selon l'une quelconque des revendications 1 à 26,pour la préparation d'un anticoagulant.

## Revendications (Revendications pour l'(les) Etat(s) contractant(s) suivant(s): GR)

1. Dérivé de peptide alcool formule
X-A₁-A₂-A₃-A₄-A₅-A₆-A₇-A₈-A₉-A₁₀-ol
dans laquelle
X est un hydrogène, un ou deux groupes alkyles ayant de 1 à 6 atomes de carbone, ou un ou deux groupes acyles ayant de 2 à 10 atomes de carbone ;
A₁ est une liaison ou un fragment peptidique contenant de 1 à 11 restes amino-acides quelconques ;
A₂ est Phe, SubPhe, β-(2- et 3-thiényl)alanine, β-(2- et 3-furannyl)alanine, β-(2-, 3- et 4-pyridyl)alanine, β-(benzothiényl-2- et -3-yl)alanine, β-(1 - et 2-naphtyl)alanine, Tyr ou Trp ;
A₃ est Glu ou Asp ;
A₄ est un amino-acide quelconque ;
A₅ est Ile, Val, Leu, Nle ou Phe ;
A₆ est Pro, Hyp, 3,4-déshydroPro, thiazolidine-4-carboxylate, Sar, NMePgl ou D-Ala ;
A₇ est un amino-acide quelconque ;
A₈ est un amino-acide quelconque ;
A₉ est un amino-acide lipophile sélectionné parmi Tyr, Trp, Phe, Leu, Nle, Ile, Val, Cha et Pro ou est un dipeptide contenant au moins un de ces amino-acides lipophiles ;
A₁₀-ol est un fragment peptidique réduit contenant de 0 à 5 restes amino-acides quelconques, dans lequel l'amino-acide carbone-terminal est réduit en son dérivé alcool, ou ol, ou
un de ses sels pharmaceutiquement acceptables.

2. Dérivé de peptide alcool de la revendication 1, dans lequel A₂ est Phe, β-(2- ou 3-thiényl)alanine, ou Tyr.

3. Dérivé de peptide alcool de la revendication 1 ou 2, dans lequel A₃ est Glu.

4. Dérivé de peptide alcool d'une quelconque des revendications 1 à 3, dans lequel A₄ est Glu, Ala ou Pro.

5. Dérivé de peptide alcool d'une quelconque des revendications 1 à 4, dans lequel A₅ est Ile.

6. Dérivé de peptide alcool d'une quelconque des revendications 1 à 5, dans lequel A₆ est Pro.

7. Dérivé de peptide alcool d'une quelconque des revendications 1 à 6, dans lequel A₇ est Glu ou Ala.

8. Dérivé de peptide alcool d'une quelconque des revendications 1 à 7, dans lequel A₈ est Glu ou Asp.

9. Dérivé de peptide alcool d'une quelconque des revendications 1 à 8, dans lequel A₉ est Tyr-Leu, Ala-Tyr ou Ala-Cha.

10. Dérivé de peptide alcool d'une quelconque des revendications 1 à 9, dans lequel A₁₀-ol est Gln-ol, Asp-ol, Pro-ol, Asn-ol, Asp-Glu-ol, Glu-ol. Ala-ol, D-Lys-ol, Lys-ol, D-Asp-ol, D-Glu-ol ou Orn-ol.

11. Dérivé de peptide alcool d'une quelconque des revendications 1 à 10, dans lequel X est H, acétyle ou succinyle.

12. Dérivé de peptide alcool d'une quelconque des revendications 1 à 11, dans lequel A₁ est
Ser-His-Asn-Asp-Gly-Asp,
Asn-Asp-Gly-Asp,
Asp-Gly-Asp,
Gly-Asp,
Asp, ou
une liaison.

13. Dérivé de peptide alcool d'une quelconque des revendications 1 à 11, dans lequel A₁ est -Thr-Pro-Lys-Pro-Gln-Ser-His-Asn-Asp-Gly-Asp-.

14. Dérivé de peptide alcool d'une quelconque des revendications 1 à 11, dans lequel A₁ est -Thr-Pro-Asn-Pro-Glu-Ser-His-Asn-Asn-Gly-Asp-.

15. Dérivé de peptide alcool de la revendication 1, qui est H-Gly-Asp-Phe-Glu-Glu-Ile-Pro-Glu-Glu-Tyr-Leu-Gln-ol.

16. Dérivé de peptide alcool de la revendication 1, qui est Suc-Phe-Glu-Glu-Ile-Pro-Glu-Glu-Tyr-Leu-Gln-ol.

17. Dérivé de peptide alcool de la revendication 1, qui est H-Gly-Asp-Phe-Glu-Pro-Ile-Pro-Glu-Asp-Ala-Tyr-Asp-Glu-ol.

18. Dérivé de peptide alcool de la revendication 1, qui est Suc-Phe-Glu-Pro-Ile-Pro-Glu-Glu-Tyr-Leu-Pro-ol.

19. Dérivé de peptide alcool de la revendication 1, qui est Suc-Phe-Glu-Glu-Ile-Pro-Glu-Glu-Tyr-Leu-Gln-ol.

20. Dérivé de peptide alcool de la revendication 1, qui est Suc-Phe-Glu-Pro-Ile-Pro-Glu-Glu-Tyr-Leu-Gln-ol.

21. Dérivé de peptide alcool de la revendication 1, qui est Suc-Tyr-Glu-Pro-Ile-Pro-Glu-Glu-Ala-Phe-D-Lys-ol.

22. Dérivé de peptide alcool de la revendication 1, qui est Suc-Tyr-Glu-Pro-Ile-Pro-Glu-Glu-Ala-Tyr-Lys-ol.

23. Dérivé de peptide alcool de la revendication 1, qui est Suc-Tyr-Glu-Pro-Ile-Pro-Glu-Glu-Ala-Cha-Lys-ol.

24. Dérivé de peptide alcool de la revendication 1, qui est Suc-Tyr-Glu-Pro-Ile-Pro-Glu-Glu-Ala-Phe-Glu-ol.

25. Dérivé de peptide alcool de la revendication 1, qui est Suc-Tyr-Glu-Pro-Ile-Pro-Glu-Glu-Ala-Phe-Gln-ol.

26. Dérivé de peptide alcool de la revendication 1, qui est Suc-Tyr-Glu-Pro-Ile-Pro-Glu-Glu-Ala-Cha-Asp-ol.

27. Procédé pour la préparation d'un dérivé de peptide alcool selon une quelconque des revendications 1 à 26 qui comprend
(a) la liaison d'un amino-acide convenablement protégé correspondant à l'amino-acide A₉ à un support en résine activé.
(b) suivie de la liaison des autres amino-acides dont le groupe alpha-amino est protégé, dans l'ordre allant de l'extrémité carboxy à l'extrémité amino, de A₈ à A₁, à l'extrémité amino de la chaîne peptidique en construction, qui a été dans l'intervalle exposée par élimination de son groupement protecteur de l'amino ; et
(c) enfin le traitement du peptide lié à la résine par environ deux équivalents de l'amino-alcool carboxy-terminal correspondant à A₁₀-ol et environ 1 équivalent d'acide acétique dans le dichlorométhane pour libérer le peptide de la résine et simultanément lier l'alcool de peptide carboxy-terminal au peptide, formant ainsi le dérivé de peptide alcool voulu.

28. Dérivé de peptide d'une quelconque des revendications 1 à 26 ou un de leurs mélanges, pour leur utilisation en tant que substance pharmaceutiquement active.

29. Dérivé de peptide d'une quelconque des revendications 1 à 26 ou un de leurs mélanges, pour leur utilisation en tant qu'anticoagulant.
